# EUROPEAN PATENT APPLICATION

(11) **EP 4 741 040 A1**
(43) Date of publication of application: **13.05.2026**
(21) Application number: 24835122.3
(22) Date of filing: 08.05.2024
(51) Int. Cl.: B01D 47/16, B01D 47/18, B01D 47/06, C07C 253/34

(54) **ABSORPTION DEVICE HAVING OPPOSITE SPRAY LIQUID INLETS AND MANUFACTURING METHOD FOR NITRILE**

(30) Priority: 05.07.2023 CN 202310819832; 05.07.2023 CN 202310819780
(71) Applicant: China Petroleum & Chemical Corporation, Beijing 100728 (CN); Sinopec (Shangai) Research Institute of Petrochemical Technology Co., Ltd., Shanghai 201208 (CN)
(72) Inventor: ZHAO, Le, Shanghai 201208 (CN); WU, Lianghua, Shanghai 201208 (CN)
(74) Representative: karo IP
(86) International application number: PCT/CN2024/091575
(87) International publication number: WO 2025/007637

(57) **Abstract**

The present invention relates to an absorption device with opposing-placed spraying liquid inlets and a process for producing nitrile. The absorption device can maximize the ammonia absorption rate and reduce ammonia breakthrough. The absorption device of the present invention comprises a shell and a plurality of spraying devices arranged in layers along the central axis direction of the absorption device at predetermined vertical spacings, wherein each of the spraying devices independently comprises a spraying liquid inlet, a first spraying pipe in fluid communication with the spraying liquid inlet, a plurality of second spraying pipes in fluid communication with the first spraying pipe and, perpendicularly to the first spraying pipe, arranged along both sides thereof, a plurality of third spraying pipes in fluid communication with the second spraying pipes and, perpendicularly to the second spraying pipes, arranged along both sides thereof, and nozzles located at the ends of the third spraying pipes and in fluid communication therewith, wherein when a cross-section is obtained by cutting the absorption device at a direction perpendicular to the central axis of the absorption device, at least one selected from the group consisting of the first spraying pipe, the second spraying pipes, and the third spraying pipes of one of the plurality of spraying devices and at least one selected from the group consisting of the first spraying pipe, the second spraying pipes, and the third spraying pipes of another of the plurality of spraying devices substantially coincide in terms of the projection on the cross-section, and the angle between the projections of the spraying liquid inlet of the one spraying device and the spraying liquid inlet of the another spraying device on the cross-section is 180°.

## Description

### TECHNICAL FIELD

The present invention relates to the technical field of gas absorption, and more particularly, to an absorption device with opposing-placed spraying liquid inlets and a process for producing nitrile.

### BACKGROUND

In the process route for producing corresponding nitriles by ammoniation or ammoxidation, in order to maximize the conversion of feedstock gases such as hydrocarbons, the feedstock gas ammonia is generally used in an excess amount, i.e., the molar ratio of ammonia to hydrocarbon feedstock gas being greater than 1. For example, in ammoxidation of propylene, the ammonia ratio (molar ratio of ammonia to propylene) is 1.10-1.35, and in ammoxidation of aromatic hydrocarbons, the ammonia ratio (molar ratio of ammonia to aromatic hydrocarbons) is 4-8. Therefore, the reactor outlet tail gas necessarily contains unreacted ammonia. On one hand, as in acrylonitrile production processes, reaction gases such as acrylonitrile are prone to polymerization under alkaline conditions. On the other hand, the escape of even a small amount of unreacted ammonia can easily cause environmental pollution. Therefore, in ammonification or ammoxidation processes, it is desired to use an absorption device (generally called as an ammonia absorption column or quench column) to remove unreacted ammonia from the gas phase using acid or water, which process is very necessary..

With the development of production technology, production loads are continuously increasing, and the trend towards large-scale of devices represents the future development direction. The higher the device load, the larger the equipment, including the absorption device. It is known that in the absorption device, the circulating liquid (spraying liquid) is distributed within the absorption device via a spraying device and contacted countercurrently with the ammonia-containing gas to be absorbed, achieving the purpose of removing residual ammonia from the gas phase.

CN105425849 teaches removal of residual ammonia by adjusting the amount of acid added based on the pH value of the effluent from the absorption device. CN1199940 teaches to improve the mass transfer and heat transfer effect between gas and liquid phases by adding internal components at the bottom of the absorption device, which in fact addresses the issue of uniform distribution of the ammonia-containing gas phase. However, the absorption device is still inevitably subjected to ammonia breakthrough, i.e., a small amount of ammonia escape still existing, leading to product loss in subsequent refining and separation units or causing environmental pollution.

In the ammonia absorption methods of the prior art, after long-term operation of the absorption device, the ammonia content in the absorption tail gas increases significantly compared with the initial period of the operation.

### SUMMARY OF THE INVENTION

The inventors of the present invention have found that, as the spraying liquid inlets of existing devices are generally arranged on the same side, after long operation cycles, solid impurities or other viscous heavy components and other deposition-prone substances accumulate in the circulating spraying liquid, which adhere to the inner walls of the spraying pipelines and nozzles of the spraying device, causing the fluid flow resistance to continuously increase, resulting in insufficient nozzle pressure and poor atomization effect. The farther the distance away from the spraying liquid inlet, the more severe the accumulation problem. After long-term operation, the degree of accumulation at different positions of the spraying device varies, leading to different atomization degrees of the spraying liquid from different nozzles. In regions with poor atomization, ammonia escape becomes significant.

The inventors of the present invention, by analyzing the accumulation degrees of easily deposition-prone substances at various parts of the spraying device as the operation cycle continues and its impact on the atomization effect, and comprehensively considering the complementarity between regions with different atomization degrees (different degrees of deterioration) of different spraying devices, have discovered that by arranging a plurality of layers of spraying devices and making the vertical projections of different spraying devices overlap and the spraying liquid inlets opposing-placed to each other, regions with different atomization degrees can correspond to and compensate for each other, achieving overall uniform atomization degree.

The inventors of the present invention have also found that, as the spraying liquid in the prior art is ejected with the same rotating direction, during the downward movement of the spraying liquid, droplets formed by one spraying liquid are contacted with droplets formed by another spraying liquid, causing the droplet size to increase, which reduces the effective area for gas-liquid contact, leading to lower acid utilization efficiency, accompanied by more ammonia escape.

The inventors of the present invention have found that this problem can be solved by arranging the spraying liquid inlet of one spraying device horizontally opposing-placed to that of another spraying device, and further preferably by, for example, controlling the rotational ejection direction of the spraying liquid from two adjacent nozzles. The present invention is completed based on this discovery.

Specifically, the present invention relates to the following aspects.
1. An absorption device, comprising a shell and a plurality (e.g., 2-10, preferably 4-8) of spraying devices arranged in layers along the central axis direction of the absorption device at predetermined vertical spacings, wherein each of the spraying devices independently comprises a spraying liquid inlet, a first spraying pipe in fluid communication with the spraying liquid inlet, a plurality (e.g., 10-26, preferably 12-22) of second spraying pipes in fluid communication with the first spraying pipe and, perpendicularly to the first spraying pipe, arranged along both sides thereof, a plurality (e.g., 4-26, preferably 6-22) of third spraying pipes in fluid communication with the second spraying pipes and, perpendicularly to the second spraying pipes, arranged along both sides thereof, and nozzles located at the ends of the third spraying pipes and in fluid communication therewith, wherein when a cross-section is obtained by cutting the absorption device perpendicular to the central axis direction of the absorption device, at least one (preferably all) selected from the group consisting of the first spraying pipe, the second spraying pipes, and the third spraying pipes of one of the plurality of spraying devices and at least one (preferably all) selected from the group consisting of the first spraying pipe, the second spraying pipes, and the third spraying pipes of another of the plurality of spraying devices substantially coincide in terms of the projection on the cross-section, and the angle between the projection of the spraying liquid inlet of the one spraying device and the projection of the spraying liquid inlet of the another spraying device on the cross-section is 180°.
2. The absorption device according to any preceding or subsequent aspect, wherein the plurality of second spraying pipes extend substantially in parallel in the horizontal direction perpendicularly to the first spraying pipe towards its opposite two sides, and/or, the plurality of third spraying pipes extend substantially in parallel in the horizontal direction perpendicularly to the second spraying pipes towards their opposite two sides.
3. The absorption device according to any preceding or subsequent aspect, wherein the first spraying pipe has an inner diameter of 160-480 mm (preferably 200-450 mm), and a length of 4500-11500 mm (preferably 4800-10500 mm), and/or, the plurality of second spraying pipes are the same or different from each other, each independently has an inner diameter of 30-150 mm (preferably 40-120 mm), and a length of 1200-5750 mm (preferably 1800-5250 mm), and/or, the plurality of third spraying pipes are the same or different from each other, each independently has an inner diameter of 10-60 mm (preferably 15-50 mm), and a length of 160-325 mm (preferably 175-300 mm).
4. The absorption device according to any preceding or subsequent aspect, wherein the nozzles are the same or different from each other, each independently having an inner diameter (referring to the nozzle outlet) of 3-20 mm (preferably 6-14 mm), and a spray angle of 65-120° (preferably 70-100°).
5. The absorption device according to any preceding or subsequent aspect, wherein on the first spraying pipe, the horizontal spacing between two adjacent second spraying pipes is 640-1300 mm (preferably 700-1200 mm), and/or, on a same second spraying pipe, the horizontal spacing between two adjacent third spraying pipes is 320-650 mm (preferably 350-600 mm), and/or, on two adjacent second spraying pipes, the linear distance, M, between the end of any one third spraying pipe on one second spraying pipe and the end of any one third spraying pipe on another adjacent second spraying pipe is not less than 320 mm (preferably not less than 350 mm).
6. The absorption device according to any preceding or subsequent aspect, wherein the nozzles are the same or different from each other, each independently having a spraying liquid ejection rate of 0.5-7.5 t/h (preferably 0.9-6.5 t/h).
7. The absorption device according to any preceding or subsequent aspect, wherein among all the spraying devices, the angle between the projections, on the cross-section, of the spraying liquid inlets of any two odd-numbered spraying devices is 0°, the angle between the projections, on the cross-section, of the spraying liquid inlets of any two even-numbered spraying devices is 0°, and the angle between the projections, on the cross-section, of the spraying liquid inlet of any odd-numbered spraying device and of the spraying liquid inlet of any even-numbered spraying device is 180°.
8. The absorption device according to any preceding or subsequent aspect, wherein the absorption device has an inner diameter of 4.5-11.5 m (preferably 4.8-10.5 m).
9. The absorption device according to any preceding or subsequent aspect, wherein all the nozzles of the one spraying device and of the another spraying device substantially coincide in terms of the projection on the cross-section, and/or, two nozzles with substantially coinciding projections have the same spray diameter.
10. The absorption device according to any preceding or subsequent aspect, wherein the nozzle comprises a nozzle inlet, a rotating chamber, and a nozzle outlet, wherein the rotating chamber is configured such that the spraying liquid fed in through the nozzle inlet leaves the nozzle outlet in a rotating manner after passing through the rotating chamber.
11. The absorption device according to any preceding or subsequent aspect, wherein the rotating chambers each independently have a diameter of 10.0-55.0 mm (preferably 13.0-45.0 mm), and/or, two nozzles with substantially coinciding projections have the same spraying liquid rotating direction.
12. The absorption device according to any preceding or subsequent aspect, wherein on at least one (preferably all) of the second spraying pipes, two adjacent (preferably all) nozzles located on the same side of the second spraying pipe are configured such that the spraying liquid is ejected out with the same rotating direction.
13. The absorption device according to any preceding or subsequent aspect, wherein all nozzles toward the facing sides of two side-by-side adjacent second spraying pipes are configured such that the spraying liquid is ejected out with opposite rotating directions.
14. The absorption device according to any preceding or subsequent aspect, wherein on at least one (preferably all) of the second spraying pipes, at least one (preferably all) nozzle located on one side of the second spraying pipe is configured such that the spraying liquid is ejected out with a rotating direction A, while at least one (preferably all) nozzle located on the opposite side of the second spraying pipe is configured such that the spraying liquid is ejected out with a rotating direction B, wherein the rotating direction A is opposite to the rotating direction B.
15. The absorption device according to any preceding or subsequent aspect, wherein the rotating direction A is clockwise, while the rotating direction B is counterclockwise.
16. The absorption device according to any preceding or subsequent aspect, wherein among all the nozzles of the spraying device, the number of nozzles ejecting the spraying liquid with the rotating direction A is equal or substantially equal to the number of nozzles ejecting the spraying liquid with the rotating direction B.
17. The absorption device according to any preceding or subsequent aspect, wherein the vertical spacing between two adjacent spraying devices (calculated as the vertical spacing of the spraying liquid inlets of the spraying devices) is 650-1350 mm (preferably 750-1200 mm).
18. A process for producing a nitrile, comprising a step of subjecting a hydrocarbon feedstock to ammoxidation reaction to produce a reaction product containing nitrile (called as reaction step), and a step of spraying a spraying liquid onto the reaction product to cool the reaction product (called as cooling step), wherein the spraying liquid is sprayed onto the reaction product in the absorption device according to any preceding or subsequent aspect.
19. The process according to any preceding or subsequent aspect, wherein in the cooling step, the spraying liquid and the reaction product are contacted in a countercurrent manner.
20. The process according to any preceding or subsequent aspect, wherein in the cooling step, the flow ratio of the spraying liquid to the reaction product is 15-25:1.
21. The process according to any preceding or subsequent aspect, wherein in the reaction step, the hydrocarbon feedstock is propylene, the molar ratio of propylene/ammonia/air (calculated as molecular oxygen) is 1:1.1-1.3:1.8-2.0, and the reaction involves a reaction temperature of 420-440°C, a reaction pressure (gauge pressure) of 0.03-0.14 MPa, and a catalyst weight hourly space velocity of 0.06-0.15 h⁻¹, or alternatively, the hydrocarbon feedstock is isobutylene, the molar ratio of isobutylene/ammonia/air (calculated as molecular oxygen) is 1:1.3-1.6:2.2-2.8, and the reaction involves a reaction temperature of 395-420°C, a reaction pressure (gauge pressure) of 0.03-0.14 MPa, and a catalyst weight hourly space velocity of 0.08-0.17 h⁻¹.
22. The process according to any preceding or subsequent aspect, wherein in the cooling step, the spraying liquid cools the temperature of the reaction product from 195-235°C to 81-86°C, and/or, in the cooling step, the spraying liquid reduces the ammonia content of the reaction product to 150 ppm or less.

### TECHNICAL EFFECTS

According to the present invention, even after long-term operation (e.g., continuous operation for 18 months or even longer), the ammonia content in the absorption tail gas does not increase significantly compared with the initial period of the operation, and good ammonia absorption effect can be maintained for a long period, reducing ammonia escape.

According to the present invention, even after long-term operation (e.g., continuous operation for 18 months or even longer), the total acid consumption can still be maintained at a low level, with a small increase (e.g., less than 3%).

According to the present invention, gas-liquid contact is sufficient, the ammonia absorption effect is excellent, and the amount of acid used can be reduced.

According to the present invention, the acidic circulating liquid can neutralize more ammonia in the gas phase, reducing ammonia escape from the ammonia absorption column.

### BRIEF DESCRIPTION OF THE DRAWINGS

Fig.1 is a schematic front view of an ammonia absorption column of the prior art.
Fig.2 is a schematic front view of an ammonia absorption column of the prior art.
Figs.3A and 3B are schematic front views of an ammonia absorption column of the present invention.
Figs.4A and 4B are schematic front views of an ammonia absorption column of the present invention.
Fig.5 is a schematic top view of a spraying device of the present invention.
Figs.6A and 6B are schematic top views of a spraying device of the present invention.
Figs.7A and 7B are schematic top views of a spraying device of the present invention.
Figs.8A and 8B are schematic top views of a spraying device according to Comparative Examples.
Figs.9A and 9B are schematic top and front views of a nozzle of the prior art.

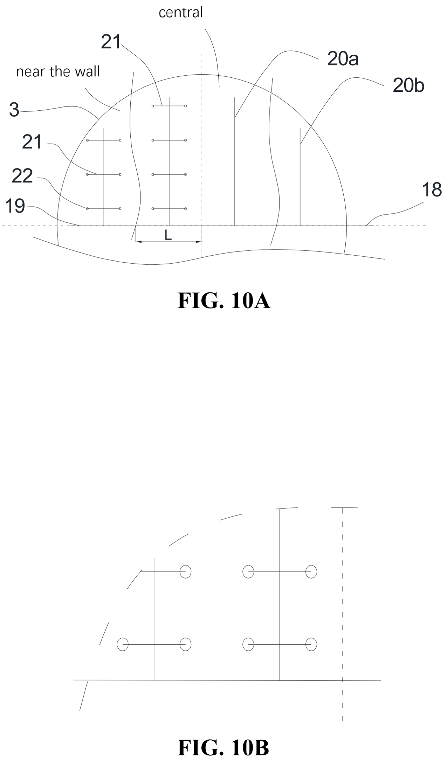

Figs.10A and 10B are a schematic top view and a detailed top view of a spraying device of the prior art.
Fig.11A is a schematic top/front view of two rotation modes of a nozzle of the present invention.
Fig.11B is a schematic top view of a spraying device.
Fig.11C is a detailed view of a schematic top view of one spraying device of the present invention.
Fig.11D is a detailed view of a schematic top view of another spraying device of the present invention.
Fig.11E is a detailed view of a schematic top view of another spraying device of the present invention.

### Illustration of reference signs:

1: Ammonia absorption column
2: An internal component, demister, of ammonia absorption column
3: An internal component, spraying device, of ammonia absorption column, 3a-3f being spraying devices
4: An internal component, gas distributor, of ammonia absorption column
5: An internal component, spraying device, of ammonia absorption column, 5a-5b being spraying devices
6: Upper section circulation pump
7: Lower section circulation pump
8: Ammonia-containing gas feed
9: Gas phase output of ammonia absorption column
10: Upper section water supplement
11: Lower section wastewater output
12: Upper section ammonium salt-containing solution output
13: Lower section circulating liquid
14: Upper section circulating liquid
15: Acid-containing solution
16: Circulating liquid
17: Circulation pump
18: Spraying device inlet
19: First spraying pipe of spraying device
20a, 20b: Second spraying pipes of spraying device
21: Third spraying pipe of spraying device
22: Atomizing nozzle of spraying device

### Embodiments

The specific embodiments of the present invention will be described in detail below, but it should be pointed out that the protection scope of the present invention is not limited by these specific embodiments, but is determined by the claims attached.

All publications, patent applications, patents, and other references mentioned in this specification are herein incorporated by reference in their entirety. Unless defined otherwise, all technical and scientific terms used herein are understood same as the meanings commonly known to those skilled in the art. In case of conflict, definitions according to the present specification will control.

When the specification introduces materials, substances, processes, steps, devices, components, or the like initiated with "known to those ordinary skill in the art", "prior art", or the like, it is intended that the subject matter so initiated encompass not only those conventionally used in the art at the time of filing this application, but also those may not be so commonly used at the present time, but will become known in the art as being suitable for a similar purpose.

In the context of this specification, the term "substantially" means a deviation not more than 20%, preferably not more than 10% or 5%.

All percentages, parts, ratios, and the like referred to in the specification are based on weight and pressures are gauge pressures unless explicitly indicated.

In the context of the present invention, any two or more embodiments or aspects of the present invention may be arbitrarily combined, and the technical solutions resulted are part of the original disclosure of the present specification, and also fall within the scope of the present invention.

In the context of this specification, all technical details not mentioned, etc., directly apply the relevant information known in the art.

According to one embodiment of the present invention, an absorption device is provided, particularly an ammonia absorption column or a quench column.

According to one embodiment of the present invention, the absorption device comprises a shell and a plurality (e.g., 2-10, preferably 4-8) of spraying devices arranged in layers along the central axis direction of the absorption device at predetermined vertical spacings.

According to one embodiment of the present invention, each of the spraying devices independently comprises a spraying liquid inlet, a first spraying pipe in fluid communication with the spraying liquid inlet, a plurality (e.g., 10-26, preferably 12-22) of second spraying pipes in fluid communication with the first spraying pipe and, perpendicularly to the first spraying pipe, arranged along both sides thereof, a plurality (e.g., 4-26, preferably 6-22) of third spraying pipes in fluid communication with the second spraying pipes and, perpendicularly to the second spraying pipes, arranged along both sides thereof, and nozzles located at the ends of the third spraying pipes and in fluid communication therewith.

The connection means between the various spraying pipes and between the third spraying pipes and the nozzles, etc. is not specifically limited by the present invention, and conventional connection means in the art can be adopted. For example, fixed connections or detachable connections can be used, preferably threaded connections, or other detachable connection means, without specific limitation.

According to one embodiment, the spraying liquid is water or an acidic aqueous solution. Preferably, the ammonia-containing gas, from bottom to top, is contacted countercurrently with the acidic aqueous solution as the spraying liquid flowing from top to bottom, and the acidic H⁺ contained in the aqueous solution neutralizes ammonia to remove it. Here, the acidic aqueous solution is an aqueous solution of an acidic substance. The acidic substance can be an inorganic acid, such as hydrochloric acid, sulfuric acid, phosphoric acid; or can also be an organic acid, such as acrylic acid, acetic acid; or can also be an acidic salt, such as ammonium sulfate, without specific limitation.

According to the present invention, the atomized droplet of the spraying liquid ejected from the nozzle has a very small droplet size, usually only 50-5000 µm, which can effectively achieve the gas absorption function, especially the ammonia removal function.

According to one embodiment of the present invention, all the nozzles of the one spraying device and of the another spraying device substantially coincide in terms of the projection on the cross-section. The inventors of the present invention have found that the plurality of spraying devices in the absorption device are not only relatively independent individuals but also integrated into a whole. The circulating liquid is delivered to the nozzles of each spraying device via the first spraying pipe, second spraying pipes, and third spraying pipes of each spraying device, and forms a hollow conical liquid surface centered on the nozzle. The gas is contacted with the circulating liquid counter-currently. The gas must pass through the conical liquid surface formed by a lower layer spraying device to be contacted with the conical liquid surface formed by the next upper layer spraying device. At the moment the gas passes through the conical liquid surface, the ammonia in the gas neutralizes with the acid in the liquid. As the ammonia-containing gas passes through a plurality of hollow conical liquid surfaces of a plurality of spraying devices, it is eventually completely neutralized by the acid in the circulating liquid. The space between the hollow conical liquid surfaces of the two layers, i.e., a upper layer and a lower layer, of the spraying devices can be regarded as a gas rising channel. As each stage requires an independent liquid phase circulation spraying device, the rising gas channel has a certain height. The projections of at least one (preferably all) of the first spraying pipe, the second spraying pipes, and the third spraying pipes on the cross-section substantially coincide, meaning that the projections of the nozzles (cone centers) of each layer coincide on the cross-section. Thus, the distribution of ammonia in the gas channels is also uniform. If the projections of the nozzles of the upper layer and of the lower layer do not coincide on the cross-section, as the hollow conical liquid surfaces of the upper and lower layers are not at the same position, the gas rising channel is changed, then after the rising gas passes through such a gas channel, gas distribution unevenness is likely to occur between the gas channels, reducing the ammonia absorption effect. According to one embodiment of the present invention, the vertical spacing between two adjacent spraying devices (calculated as the vertical spacing of the spraying liquid inlets of the spraying devices) is 650-1350 mm, preferably 750-1200 mm.

The inventors of the present invention have found that if the projections of the nozzles of the upper layer and of the lower layer do not coincide on the cross-section, as the hollow conical liquid surfaces of the upper and lower layers are not at the same position, the gas rising channels are changed, with some channels becoming "wider" and some others becoming "narrower". After the rising gas passes through gas channels of different heights, due to different gas residence times within the channels, gas distribution unevenness within the channels is likely to occur. That is, when the ammonia-containing gas passes through the hollow conical liquid surfaces, it results in excess acid in some regions of the liquid surface, while in some other regions of the liquid surface, insufficient acid leads to ammonia breakthrough, thereby reducing the ammonia absorption effect.

The inventors of the present invention have also found that if the projections of the nozzles of the upper layer and of the lower layer do not coincide on the cross-section, the spraying liquid ejected from the upper layer nozzles and that from the lower layer nozzles are subjected to more collisions. When two droplets collide, behaviors such as separation, coalescence, breakup, etc., can occur. The inventors have found that when the projections of the nozzles of the upper layer and of the lower layer do not coincide, droplets generated by the upper layer nozzles and droplets generated by the lower layer nozzles are more likely to coalesce during collision, forming larger droplets, which is unbeneficial to ammonia absorption.

According to one embodiment of the present invention, when a cross-section is obtained by cutting the absorption device at a direction perpendicular to the central axis of the absorption device, at least one (preferably all) selected from the group consisting of the first spraying pipe, the second spraying pipes, and the third spraying pipes of one of the plurality of spraying devices and at least one (preferably all) selected from the group consisting of the first spraying pipe, the second spraying pipes, and the third spraying pipes of another of the plurality of spraying devices substantially coincide in terms of the projection on the cross-section. That is, the projections of the nozzles (cone centers) of each layer coincide on the cross-section, thus, the distribution of ammonia in the gas channels is uniform.

The inventors of the present invention have also found that the circulating liquid in each spraying device flows along the first spraying pipe to the second spraying pipes and then to the third spraying pipes until the nozzles. During the fluid flow, the pressure continuously drops along the path due to the influence of pipe wall resistance. The pressure at the nozzle farthest away from the spraying liquid inlet is lower than the pressure at the nozzle closest to the spraying liquid inlet. As the pressure at the farthest nozzle is relatively the lowest, the atomization effect is also relatively the poorest, leading to insufficient gas-liquid contact at the farthest nozzle, which easily causes ammonia escape. As the operation cycle of the device prolongs, e.g., the device is operated continuously for 18 months or even longer, solid impurities and viscous heavy components adhere to the inner wall of the pipes, further increasing the pipe wall resistance. The atomization effect at the farthest nozzle becomes even worse, causing more ammonia escape. If the spraying liquid inlets of the plurality of layers of spraying devices are on a same side, as the farthest nozzles of each layer of spraying device are on the same side, the gas-liquid contact in this area is the weakest, and ammonia in the gas phase is more likely to escape from the ammonia absorption column from this area.

Based on such discovery, according to one embodiment of the present invention, the angle between the projections of the spraying liquid inlet of the one spraying device and the spraying liquid inlet of the another spraying device on the cross-section is 180°. At the initial period of the operation of the device, as the pipelines of the spraying device are relatively clean, the farthest nozzles of the spraying device are sufficient to atomize the spraying liquid to produce droplets of 50-5000 µm or smaller. However, after the device operates continuously for 18 months or even longer, although at the farthest nozzle of one spraying device, the poor atomization effect of the nozzle deteriorate, leading to ammonia escape caused by poor gas-liquid contact, the escaping ammonia is captured by the spraying liquid from the near nozzles of the upper layer spraying device and undergoes a neutralization reaction to form the corresponding salt. Usually, a plurality of layers of spraying devices are arranged in the ammonia absorption column, such as 3 layers, 4 layers, 5 layers or more. When the projection angle between the spraying liquid inlets of the upper one (or two) layer(s) of spraying device(s) and the lower one (or two) layer(s) of spraying device(s) on the cross-section is 180°, ammonia can be absorbed to a greater extent, thereby reducing ammonia escape and also reducing acid consumption to a greater extent.

According to one embodiment of the present invention, the plurality of second spraying pipes extend substantially in parallel in the horizontal direction perpendicularly to the first spraying pipe towards its opposite two sides.

According to one embodiment of the present invention, the plurality of third spraying pipes extend substantially in parallel in the horizontal direction perpendicularly to the second spraying pipes towards their opposite two sides.

According to one embodiment of the present invention, the first spraying pipe has an inner diameter of 160-480 mm (preferably 200-450 mm), and a length of 4500-11500 mm (preferably 4800-10500 mm).

According to one embodiment of the present invention, the plurality of second spraying pipes are the same or different from each other, each independently having an inner diameter of 30-150 mm (preferably 40-120 mm), and a length of 1200-5750 mm (preferably 1800-5250 mm).

According to one embodiment of the present invention, the plurality of third spraying pipes are the same or different from each other, each independently having an inner diameter of 10-60 mm (preferably 15-50 mm), and a length of 160-325 mm (preferably 175-300 mm).

According to one embodiment of the present invention, the nozzles are the same or different from each other, each independently having an inner diameter (referring to the nozzle outlet) of 3-20 mm (preferably 6-14 mm).

According to one embodiment of the present invention, the nozzles each independently have a spray angle of 65-120° (preferably 70-100°).

According to one embodiment of the present invention, on the first spraying pipe, the horizontal spacing between two adjacent second spraying pipes is 640-1300 mm (preferably 700-1200 mm).

According to one embodiment, on a same second spraying pipe, the horizontal spacing between two adjacent third spraying pipes is 320-650 mm (preferably 350-600 mm).

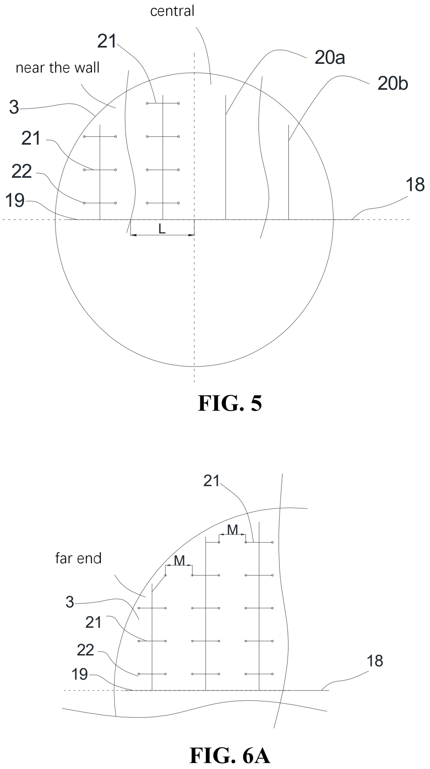

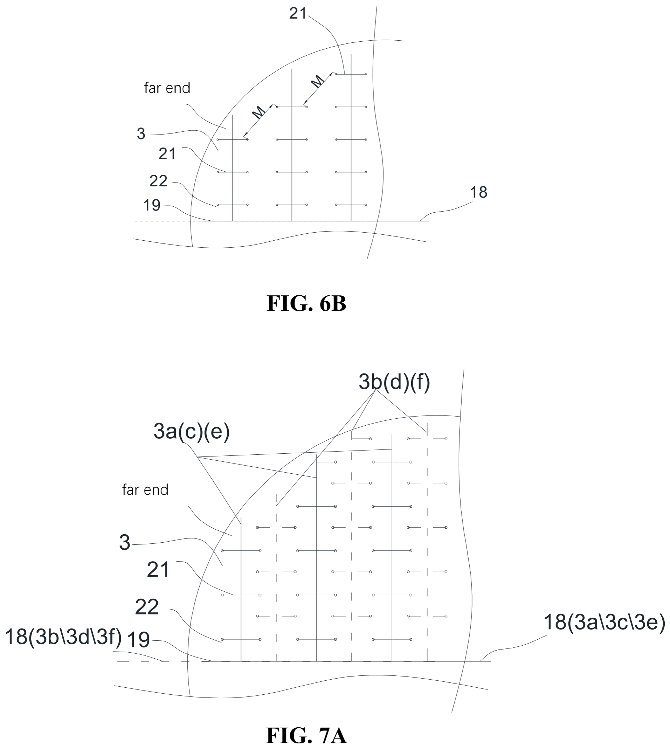

According to one embodiment, on two adjacent second spraying pipes, the linear distance, M, between the end of any one third spraying pipe on one second spraying pipe and the end of any one third spraying pipe on another adjacent second spraying pipe (as shown in Fig.6A and Fig.6B) is not less than 320 mm, preferably not less than 350 mm. The inventors of the present invention have found that in order to improve the ammonia absorption efficiency, generally, any position on the cross-section inside the column is covered by at least two or more overlapping conical liquid surfaces formed taking the nozzle as center, which is under the same situation at the column wall. If the distance between the ends of two spraying pipes is too large, limited by the nozzle structure, it is difficult to meet the requirement that any position at the column wall is covered by overlapping liquid surfaces sprayed from two or more nozzles, which increases the chance of ammonia escaping from the "gaps". If the distance between the ends of two spraying pipes is too small, in order to ensure the atomization quality of the spraying liquid, the amount of circulating liquid in the ammonia absorption column must be increased, i.e., increasing the pump energy consumption. In addition, as shown in Figs.6A and 6B, due to the overlapping vertical projections of the upper and lower layers of spraying devices, only the upper spraying device and the mutually opposing-placed spraying liquid inlets are visible.

According to one embodiment, the nozzles are the same or different from each other, each independently having a spraying liquid ejection rate of 0.5-7.5 t/h (preferably 0.9-6.5 t/h).

According to one embodiment of the present invention, among all the spraying devices, the angle between the projections, on the cross-section, of the spraying liquid inlets of any two odd-numbered spraying devices is 0°, the angle between the projections, on the cross-section, of the spraying liquid inlets of any two even-numbered spraying devices is 0°, and the angle between the projections, on the cross-section, of the spraying liquid inlet of any odd-numbered spraying device and of the spraying liquid inlet of any even-numbered spraying device is 180°. The inventors of the present invention have found that such arrangement can maximumly satisfy the uniform distribution of ammonia in the gas phase within the gas channels.

According to one embodiment of the present invention, the absorption device has an inner diameter of 4.5-11.5 m (preferably 4.8-10.5 m).

According to one embodiment of the present invention, all the nozzles of the one spraying device and of the another spraying device substantially coincide in terms of the projection on the cross-section.

According to one embodiment of the present invention, two nozzles with substantially coinciding projections have the same spray diameter.

According to one embodiment, the vertical spacing between two adjacent spraying devices (calculated as the vertical spacing between the spraying liquid inlets of the spraying devices) is 650-1350 mm (preferably 750-1200 mm). The inventors of the present invention have found that when the vertical spacing between two adjacent spraying devices is less than 650 mm, for an ammonia absorption column with the same number of spraying devices, the contact time between the rising ammonia-containing gas and the descending circulating liquid is insufficient, causing a part of the ammonia in the gas phase to directly pass through the hollow conical liquid surface formed by the circulating liquid, resulting in poor ammonia absorption efficiency. Although the number of spraying devices can be increased to meet sufficient gas-liquid contact time and achieve complete absorption of ammonia in the gas phase, this would increase the total amount of circulating liquid and increase pump energy consumption, which is obviously uneconomical. When the vertical spacing between two adjacent spraying devices is greater than 1350 mm, with the same number of spraying devices, the height of the column increases, i.e., equipment investment costs increase, and besides, maintenance and repair difficulty also increases.

According to one embodiment of the present invention, the nozzle comprises a nozzle inlet, a rotating chamber, and a nozzle outlet, wherein the rotating chamber is configured such that the spraying liquid fed in through the nozzle inlet leaves the nozzle outlet in a rotating manner after passing through the rotating chamber. According to the present invention, the rotating chamber can adopt any known rotating chamber structure in the art, as long as it can allow the spraying liquid leave the nozzle outlet in a rotating manner after passing through the rotating chamber, without particular limitation.

According to one embodiment of the present invention, the rotating chambers each independently have a diameter of 10.0-55.0 mm (preferably 13.0-45.0 mm).

According to one embodiment of the present invention, two nozzles with substantially coinciding projections have the same spraying liquid rotating direction. As the spraying liquids ejected from the coinciding projection nozzles of two spraying devices collide during descending, the droplet states of the respective nozzles are different. Relatively speaking, two nozzles with coinciding projections and opposite rotating directions are more likely to cause droplet breakage, forming a plurality of smaller droplets compared to two nozzles with the same rotating direction. However, if the droplets are too small, they are easily entrained by the gas and escape.

According to one embodiment of the present invention, on at least one (preferably all) of the second spraying pipes, two adjacent (preferably all) nozzles located on a same side of the second spraying pipe are configured such that the spraying liquid is ejected out with the same rotating direction. Usually, the spraying liquid is fed into the rotating chamber at the tangential direction of the nozzle, and after passing through the nozzle outlet, forms a hollow cone with the nozzle outlet as vertex of the cone. To achieve uniformity of the spraying liquid within the column, two adjacent nozzles are required to be equidistantly distributed on the column cross-section, i.e., the spraying liquid enters the nozzles with the same tangential direction. Therefore, the spraying liquid on a same side of the second spraying pipe is ejected out with the same rotating direction, while the spraying liquids on the opposite two sides of the second spraying pipe are ejected out with opposite rotating directions. Thus, after the spraying liquids ejected from nozzles on the same second spraying pipe coalesce and collide, more droplets maintain their original state and continue descend along the original motion direction.

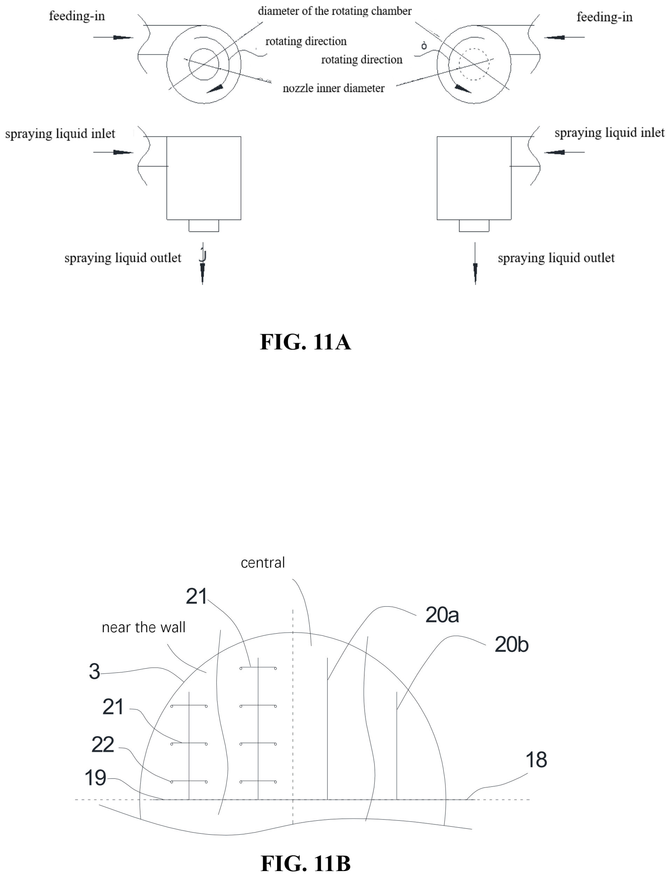

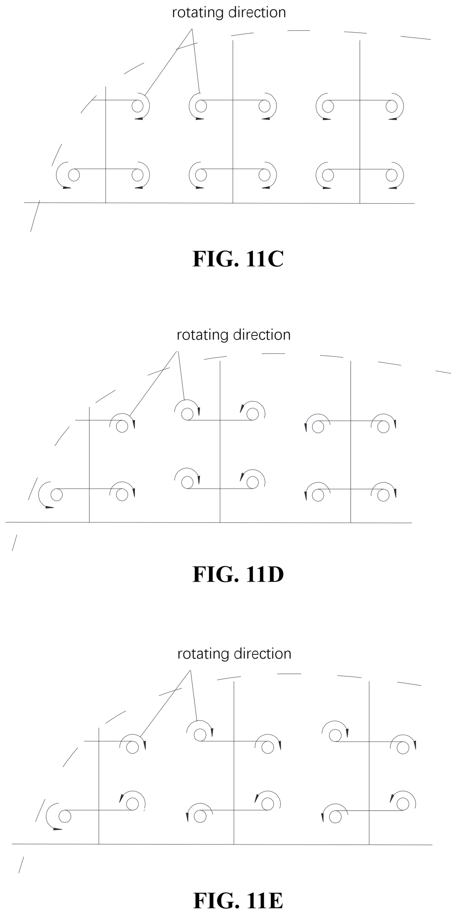

According to one embodiment of the present invention, all nozzles toward the facing sides of two side-by-side adjacent second spraying pipes are configured such that the spraying liquid is ejected out with opposite rotating directions. Here, "side-by-side adjacent" refers to being on a same side of the first spraying pipe and adjacent to each other, and "respectively toward the facing sides" refers to the respective sides of one of the second spraying pipe and of another second spraying pipe that face each other, as shown in Fig.11C. After the spraying liquids ejected from the two adjacent nozzles of side-by-side adjacent second spraying pipes collide during their respective descending processes, the droplets can still maintain their original state and continue moving downward. The droplets are not prone to coalesce into larger droplets, nor are they prone to break into finer droplets.

According to one embodiment of the present invention, on at least one (preferably all) of the second spraying pipes, at least one (preferably all) nozzle located on one side of the second spraying pipe is configured such that the spraying liquid is ejected out with a rotating direction A, while at least one (preferably all) nozzle located on the opposite side of the second spraying pipe is configured such that the spraying liquid is ejected out with a rotating direction B, wherein the rotating direction A is opposite to the rotating direction B.

According to one embodiment of the present invention, the rotating direction A is clockwise, while the rotating direction B is counterclockwise.

According to one embodiment of the present invention, among all the nozzles of the spraying device, the number of nozzles ejecting the spraying liquid with the rotating direction A is equal or substantially equal to the number of nozzles ejecting the spraying liquid with the rotating direction B. As mentioned above, the premise of uniform spraying is the uniform distribution of nozzles within the column, usually with axisymmetric distribution preferably. Therefore, the rotating directions of the nozzles appear in axisymmetric pairs. If the rotating directions of the paired nozzles are all the same, it means that all nozzles have a same rotating direction, which easily causes the spraying liquids ejected from two adjacent nozzles to collide and become larger during rotation and descending, during which the total effective contact area with gaseous ammonia decreases, such that the ammonia absorption efficiency is reduced.

According to one embodiment of the present invention, on at least one (preferably all) of the second spraying pipes, two adjacent (preferably all) nozzles located on a same side of the second spraying pipe are configured such that the spraying liquids are ejected out with opposite rotating directions. The third spraying pipes extend substantially in parallel in the horizontal direction perpendicularly to the second spraying pipes towards their opposite two sides. All nozzles on the same horizontal extension line of the second spraying pipe are configured such that the spraying liquid is ejected out with the same rotating direction, as shown in Fig.11E.

According to one embodiment of the present invention, on at least one (preferably all) of the second spraying pipes, one nozzle located on the second spraying pipe is configured such that the spraying liquid is ejected out with a rotating direction A, while at least another adjacent nozzle on the same side is configured such that the spraying liquid is ejected out with a rotating direction B. At least one (preferably all) nozzle located on the opposite side of the second spraying pipe is configured such that the spraying liquid is ejected out with a rotating direction A, wherein the rotating direction A is opposite to the rotating direction B.

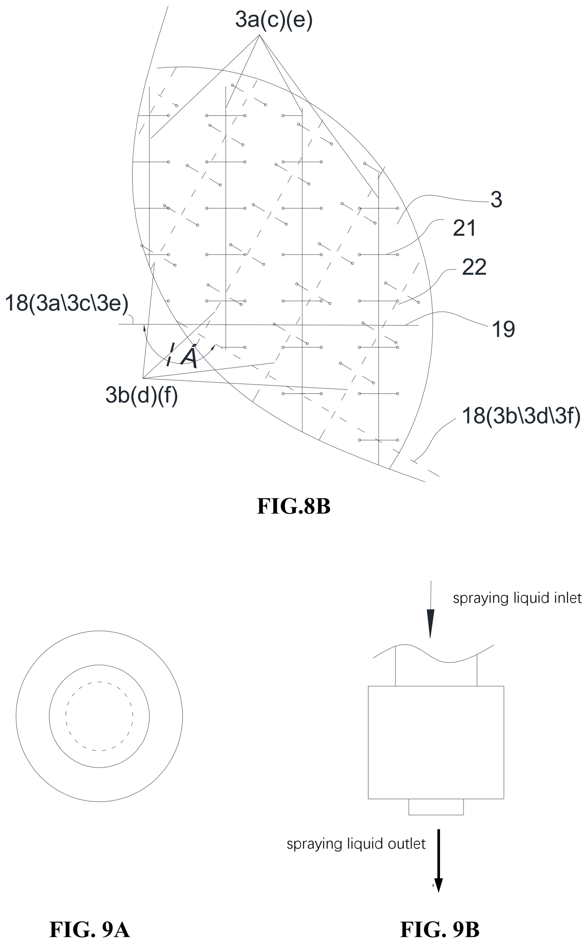

According to one embodiment, the nozzle comprises a nozzle inlet, a nozzle cavity, and a nozzle outlet, wherein the cavity has a special structure such that the spraying liquid fed in through the nozzle inlet forms droplets after passing through the cavity and leaving the nozzle outlet, as shown in Fig.9. The cavity can adopt any known structure in the art, as long as it can make the spraying liquid form droplets upon leaving the nozzle outlet, without particular limitation.

According to one embodiment, the spraying liquid is fed into the cavity from top of the nozzle, and after passing through the nozzle outlet, forms a solid cone with the nozzle outlet as the vertex. Usually, two adjacent nozzles are equidistantly distributed on the column cross-section, and the projections of the nozzles of a plurality of spraying devices substantially coincide. After the spraying liquids ejected from two adjacent nozzles collide during their respective descending processes, the droplets can still maintain their original state and continue moving downward.

According to one embodiment of the present invention, a process for producing a nitrile, particularly (meth)acrylonitrile, is also provided.

According to one embodiment of the present invention, the process for producing a nitrile comprises a step of subjecting a hydrocarbon feedstock to ammoxidation reaction to produce a reaction product containing nitrile (called as reaction step), and a step of spraying a spraying liquid onto the reaction product to cool the reaction product (called as cooling step). Here, the spraying liquid is sprayed onto the reaction product in the absorption device according to any preceding embodiment. For this part, content not described in detail here can be directly referred to the relevant content described above for the absorption device.

According to one embodiment of the present invention, in the cooling step, the spraying liquid and the reaction product are contacted in a countercurrent manner.

According to one embodiment of the present invention, in the cooling step, the flow ratio of the spraying liquid to the reaction product is 15-25:1.

According to one embodiment of the present invention, in the reaction step, the hydrocarbon feedstock is propylene, the molar ratio of propylene/ammonia/air (calculated as molecular oxygen) is 1:1.1-1.3:1.8-2.0, and the reaction involves a reaction temperature of 420-440°C, a reaction pressure (gauge pressure) of 0.03-0.14 MPa, and a catalyst weight hourly space velocity of 0.06-0.15 h⁻¹, or alternatively, the hydrocarbon feedstock is isobutylene, the molar ratio of isobutylene/ammonia/air (calculated as molecular oxygen) is 1:1.3-1.6:2.2-2.8, and the reaction involves a reaction temperature of 395-420°C, a reaction pressure (gauge pressure) of 0.03-0.14 MPa, and a catalyst weight hourly space velocity of 0.08-0.17 h⁻¹.

According to one embodiment of the present invention, depending on the various reaction steps, the composition of the reaction product comprises generally about 10-20 wt% of C₁₋₄ nitriles (e.g., acrylonitrile, etc.), about 0.1-5 wt% of C₁₋₄ oxygen-containing compounds (e.g., acrolein, etc.), about 0.1-5 wt% of O₂, about 0.1-2 wt% of ammonia, and a balance of other impurities, based on the total weight of the reaction product as 100 wt%. After preliminary cooling, the reaction product has a temperature of generally 195-235°C, and a pressure of generally 0.03-0.14 MPaG. According to the present invention, for this specific reaction product, the aforementioned technical effects of the producing process of the present invention are particularly excellent.

According to one embodiment of the present invention, in the cooling step, the spraying liquid cools the reaction product from 195-235°C to 81-86°C. In addition, preferably, the spraying liquid reduces the ammonia content of the reaction product to 150 ppm or less.

A specific embodiment of the present invention will be described in detail below by way of example with reference to the drawings.

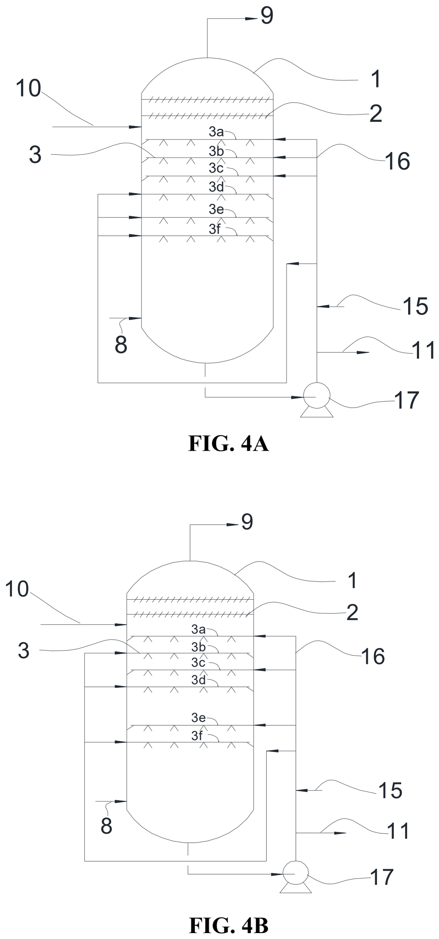

As shown in Fig.4B, according to the present invention, the reaction gas at a temperature of 225°C and unreacted ammonia are fed into the ammonia absorption column 1 through the ammonia-containing gas feeding inlet 8. The circulating liquid is withdrawn from the bottom of the column and delivered by the circulation pump 17 to spraying devices 3a-3f, wherein spraying devices 3a, 3c, 3e are placed on a same side, while spraying devices 3b, 3d, 3f are placed on the side opposing-placed to spraying devices 3a, 3c, 3e. An acid-containing liquid is added through the acid-containing solution inlet 15 to the outlet pipeline of the circulation pump. The circulating liquid is delivered through the spraying device 3, fed in through the inlet 18 of the spraying device, and passed along the fluid direction through the first spraying pipe 19, the second spraying pipes 20a (20b), and the third spraying pipes 21 to the atomizing nozzles 22. The ratio of left-rotating atomizing nozzles to right-rotating atomizing nozzles is 1:1. The circulating liquid ejected out through the nozzles 22 forms an acid mist layer in the ammonia absorption column, absorbing the gaseous ammonia from the gas feeding inlet 8. The tail gas is discharged from the gas phase outlet 9 of the ammonia absorption column. The temperature of the top tail gas is 84°C. The projections of the ends of the third spraying pipes of spraying devices 3a-3f coincide on the column cross-section, as shown in Fig.6A. The schematic diagram of the nozzle structure of the spraying device and the top view of the spraying device are shown in Figs.11C and 11B, respectively.

### EXAMPLES

The present invention will be further illustrated referring to the following Examples in more detail, but the present invention is not limited to these Examples.

### Example 1

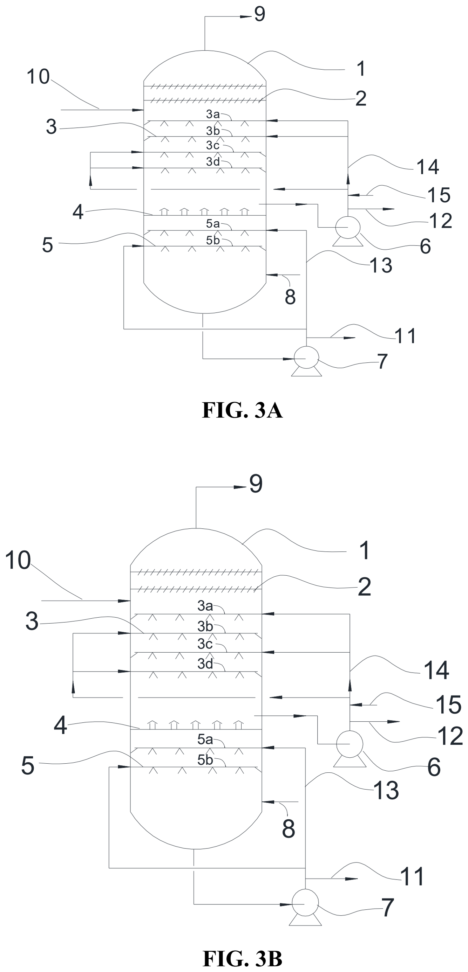

The ammonia absorption column had a two-stage structure as shown in Fig.3B. The inner diameter of the absorption column was 7200 mm. The acid-containing circulating liquid was delivered to the absorption column via an upper section circulation pump through 4 spraying devices, wherein the fluid directions in the first spraying pipes of spraying devices 3a, 3c were opposite to those of spraying devices 3b, 3d, i.e., the projection angle between two adjacent spraying liquid inlets being 180°. The top view of the spraying device was as shown in Fig.11B and the detailed top view of the spraying device was as shown in Fig.11C. The vertical spacing between two adjacent spraying devices was 1200 mm. Each spraying device had 16 second spraying pipes, and the second spraying pipes were provided with 11 to 18 third spraying pipes. The projections of the ends of the third spraying pipes of the spraying devices coincided and the rotating directions were the same. All nozzles on the same side of the second spraying pipe had the same rotating chamber direction. The nozzles on the opposite two sides of the second spraying pipe had opposite rotating chamber directions, and all nozzles toward the facing sides of two side-by-side adjacent second spraying pipes had opposite rotating directions. The numbers of nozzles with the same rotating chamber direction were 480 and 480, respectively. The vertical spacing between two adjacent spraying devices was 950 mm. The first spraying pipe of the spraying device had an inner diameter of 250 mm and a length of 7000 mm. The spacing of the second spraying pipes of the spraying device was 820 mm. The second spraying pipes had an inner diameter of 100 mm and lengths of 2100 mm-3450 mm. The spacing of the third spraying pipes of the spraying device was 410 mm. The third spraying pipes had an inner diameter of 40 mm and a length of 205 mm. The distance between the ends of two adjacent third spraying pipes as shown in Fig.6B was 580 mm. The nozzle outlet diameter was 11.5 mm, the nozzle rotating chamber diameter was 40 mm, and the spray angle of the nozzle was 75°. The vertical distance from the gas feeding inlet 8 to the spraying device 3d was 4000 mm, and the inner diameter of the feeding inlet was 1300 mm. The reaction product gas fed through the feeding inlet contained about 0.71 wt% of ammonia, 13.2 wt% of acrylonitrile, and a balance of impurities such as O₂, acrolein, nitrogen, etc., having a temperature of 225°C and a pressure of 0.06 MPaG. The spraying liquid ejection rate per nozzle was 4.8 t/h, and the weight ratio of the spraying liquid to the reaction product gas fed in through the gas inlet was 20. After 1 month of operation of the device, the residual ammonia concentration in the tail gas at the reaction outlet was 39 ppm. After 24 months of operation of the device, the residual ammonia concentration in the tail gas at the reaction outlet was 41 ppm. The ratio of acid consumption after 24 months of operation / acid consumption after 1 month of operation was 1.02.

### Example 2

The ammonia absorption column used had a single-stage structure as shown in Fig.4B. The inner diameter of the absorption column was 7200 mm. The acid-containing circulating liquid was delivered to the absorption column through 6 spraying devices via a circulation pump, wherein the fluid directions in the first spraying pipes of spraying devices 3a, 3c, 3e were opposite to those of spraying devices 3b, 3d, 3f, i.e., the projection angle between two adjacent spraying liquid inlets being 180°. The top view of the spraying device was as shown in Fig.11B and the detailed top view of the spraying device was as shown in Fig.11C. The vertical spacing between two adjacent spraying devices was 880 mm. The spraying devices each had 14 second spraying pipes, and the second spraying pipes were provided with 6 to 14 third spraying pipes. The projections of the ends of the third spraying pipes of the spraying devices coincided and the rotating directions were the same. All nozzles on the same side of the second spraying pipe had the same rotating chamber direction. The nozzles on the opposite two sides of the second spraying pipe had opposite rotating chamber directions, and all nozzles toward the facing sides of two side-by-side adjacent second spraying pipes had opposite rotating directions. The numbers of nozzles with the same rotating chamber direction were 456 and 456, respectively. The vertical spacing between two adjacent spraying devices was 920 mm. The first spraying pipe of the spraying device had an inner diameter of 200 mm and a length of 7000 mm. The spacing of the second spraying pipes was 1000 mm. The second spraying pipes had an inner diameter of 100 mm and lengths of 1850 mm-3450 mm. The spacing of the third spraying pipes was 500 mm. The third spraying pipes had an inner diameter of 40 mm and a length of 250 mm. The projections of the ends of the third spraying pipes of the spraying devices coincided. The distance between the ends of two adjacent third spraying pipes as shown in Fig.6B was 707 mm. The nozzle outlet diameter was 11.7 mm, the nozzle rotating chamber diameter was 44 mm, and the spray angle was 80°. The vertical distance from the gas feeding inlet 8 to spraying device 3d was 4000 mm, and the inner diameter of the feeding inlet was 1300 mm. The reaction product gas fed in through the feeding inlet contained about 0.71 wt% of ammonia, 13.2 wt% of acrylonitrile, and a balance of impurities such as O₂, acrolein, nitrogen, etc., having a temperature of 225°C and a pressure of 0.06 MPaG. The spraying liquid ejection rate per nozzle was 5.1 t/h, and the weight ratio of the spraying liquid to the reaction product gas fed in through the gas inlet was 20. After 1 month of operation of the device, the residual ammonia concentration in the tail gas at the reaction outlet was 34 ppm. After 24 months of operation of the device, the residual ammonia concentration in the tail gas at the reaction outlet was 37 ppm. The ratio of acid consumption after 24 months of operation / acid consumption after 1 month of operation was 1.01.

### Example 3

Example 2 was repeated, except that the ammonia absorption column used had a single-stage structure as shown in Fig.4A. The fluid directions in the first spraying pipes of spraying devices 3a, 3b, 3c were opposite to those of spraying devices 3d, 3e, 3f, i.e., the projections of the spraying inlets of the upper three layers of spraying devices 3a, 3b, 3c substantially coincided, and the projections of the spraying inlets of the lower three layers of spraying devices 3d, 3e, 3f also substantially coincided, while the projection angle between the spraying inlets of the upper three layers and those of the lower three layers was 180°. After 1 month of operation of the device, the residual ammonia concentration in the tail gas at the reaction outlet was 42 ppm. After 24 months of operation of the device, the residual ammonia concentration in the tail gas at the reaction outlet was 61 ppm. The ratio of acid consumption after 24 months of operation / acid consumption after 1 month of operation was 1.03.

### Example 4

Example 2 was repeated, except that the projections of the first spraying pipes of spraying devices 3a, 3c, 3e and those of spraying devices 3b, 3d, 3f coincided on the cross-section, while the projections of the second spraying pipes, of the third spraying pipes, and of the nozzles did not coincide on the cross-section, as shown in Fig.7A. After 1 month of operation of the device, the residual ammonia concentration in the tail gas at the reaction outlet was 78 ppm. After 24 months of operation of the device, the residual ammonia concentration in the tail gas at the reaction outlet was 95 ppm. The ratio of acid consumption after 24 months of operation / acid consumption after 1 month of operation was 1.05.

### Example 5

Example 2 was repeated, except that on each spraying device, the nozzles on the opposite two sides of 7 second spraying pipes had opposite rotating chamber directions, while the nozzles on the opposite two sides of another 7 second spraying pipes had the same rotating chamber direction. After 1 month of operation of the device, the residual ammonia concentration in the tail gas at the reaction outlet was 90 ppm. After 24 months of operation of the device, the residual ammonia concentration in the tail gas at the reaction outlet was 105 ppm. The ratio of acid consumption after 24 months of operation / acid consumption after 1 month of operation was 1.04.

### Example 6

Example 2 was repeated, except that the projections of the ends of the third spraying pipes of the spraying devices coincided and the projection-coincided nozzles of adjacent spraying devices had opposite rotating directions. After 1 month of operation of the device, the residual ammonia concentration in the tail gas at the reaction outlet was 85 ppm. After 24 months of operation of the device, the residual ammonia concentration in the tail gas at the reaction outlet was 110 ppm. The ratio of acid consumption after 24 months of operation / acid consumption after 1 month of operation was 1.05.

### Example 7

Example 2 was repeated, except that on the spraying device, two adjacent nozzles on the same side of the second spraying pipe had opposite rotating directions, while all nozzles on the extension lines at a same horizontal direction of the second spraying pipe had the same rotating direction. The top view of the spraying device was as shown in Fig.11B and the detailed top view of the spraying device was as shown in Fig.11E. After 1 month of operation of the device, the residual ammonia concentration in the tail gas at the reaction outlet was 106 ppm. After 24 months of operation of the device, the residual ammonia concentration in the tail gas at the reaction outlet was 125 ppm. The ratio of acid consumption after 24 months of operation / acid consumption after 1 month of operation was 1.05.

### Example 8

Example 2 was repeated, except that all nozzles toward the facing sides of two adjacent second spraying pipes of the spraying device had the same spraying liquid rotating direction, while the nozzles on the two sides of a same second spraying pipe had opposite spraying liquid rotating directions. After 1 month of operation of the device, the residual ammonia concentration in the tail gas at the reaction outlet was 108 ppm. After 24 months of operation of the device, the residual ammonia concentration in the tail gas at the reaction outlet was 121 ppm. The ratio of acid consumption after 24 months of operation / acid consumption after 1 month of operation was 1.04.

### Example 9

Example 2 was repeated, except that among all pairs of two adjacent nozzles on one side of the second spraying pipe of the spraying device, only one pair of two adjacent nozzles had the same rotating direction, while the other pairs of two adjacent nozzles had opposite rotating directions, and the nozzles on the opposite two sides of a same second spraying pipe had opposite rotating directions. After 1 month of operation of the device, the residual ammonia concentration in the tail gas at the reaction outlet was 125 ppm. After 24 months of operation of the device, the residual ammonia concentration in the tail gas at the reaction outlet was 148 ppm. The ratio of acid consumption after 24 months of operation / acid consumption after 1 month of operation was 1.05.

### Example 10

Example 1 was repeated, except that all nozzles of the spraying device had the same rotating chamber direction. After 1 month of operation of the device, the residual ammonia concentration in the tail gas at the reaction outlet was 129 ppm. After 24 months of operation of the device, the residual ammonia concentration in the tail gas at the reaction outlet was 149 ppm. The ratio of acid consumption after 24 months of operation / acid consumption after 1 month of operation was 1.06.

### Example 11

Example 2 was repeated, except that the spacing of adjacent third spraying pipes was 300 mm, and the distance between the ends of two adjacent third spraying pipes as shown in Fig.6A was 300 mm. The residual ammonia concentration in the tail gas at the reaction outlet was 76 ppm.

### Example 12

Example 2 was repeated, except that the distance between the ends of two adjacent third spraying pipes as shown in Fig.6A was 340 mm. The residual ammonia concentration in the tail gas at the reaction outlet was 105 ppm.

### Example 13

Example 2 was repeated, except that the vertical spacing between two adjacent spraying devices was 1500 mm, and the spacing of the third spraying pipes of the spraying device was 500 mm. After 1 month of operation of the device, the residual ammonia concentration in the tail gas at the reaction outlet was 117 ppm. After 24 months of operation of the device, the residual ammonia concentration in the tail gas at the reaction outlet was 139 ppm. The ratio of acid consumption after 24 months of operation / acid consumption after 1 month of operation was 1.05.

### Example 14

Example 2 was repeated, except that the vertical spacing between two adjacent spraying devices was 700 mm, and the spacing of the third spraying pipes of the spraying device was 500 mm. After 1 month of operation of the device, the residual ammonia concentration in the tail gas at the reaction outlet was 75 ppm. After 24 months of operation of the device, the residual ammonia concentration in the tail gas at the reaction outlet was 97 ppm. The ratio of acid consumption after 24 months of operation / acid consumption after 1 month of operation was 1.07.

### Example 15

Example 2 was repeated, except that the vertical spacing between two adjacent spraying devices was 1300 mm, and the spacing of the third spraying pipes of the spraying device was 500 mm. After 1 month of operation of the device, the residual ammonia concentration in the tail gas at the reaction outlet was 108 ppm. After 24 months of operation of the device, the residual ammonia concentration in the tail gas at the reaction outlet was 121 ppm. The ratio of acid consumption after 24 months of operation / acid consumption after 1 month of operation was 1.04.

### Example 16

Example 2 was repeated, except that the vertical spacing between two adjacent spraying devices was 550 mm. After 1 month of operation of the device, the residual ammonia concentration in the tail gas at the reaction outlet was 129 ppm. After 24 months of operation of the device, the residual ammonia concentration in the tail gas at the reaction outlet was 145 ppm. The ratio of acid consumption after 24 months of operation / acid consumption after 1 month of operation was 1.04.

### Example 17

Example 2 was repeated, except that the spraying liquid ejection rate per nozzle was 8.9 t/h, and the weight ratio of the spraying liquid to the reaction product gas fed in through the gas inlet was 35. After 1 month of operation of the device, the residual ammonia concentration in the tail gas at the reaction outlet was 85 ppm. After 24 months of operation of the device, the residual ammonia concentration in the tail gas at the reaction outlet was 99 ppm. The ratio of acid consumption after 24 months of operation / acid consumption after 1 month of operation was 1.03.

### Example 18

Example 2 was repeated, except that the spraying liquid ejection rate per nozzle was 1.6 t/h, and the weight ratio of the spraying liquid to the reaction product gas fed in through the gas inlet was 8. After 1 month of operation of the device, the residual ammonia concentration in the tail gas at the reaction outlet was 135 ppm. After 24 months of operation of the device, the residual ammonia concentration in the tail gas at the reaction outlet was 169 ppm. The ratio of acid consumption after 24 months of operation / acid consumption after 1 month of operation was 1.05.

### Example 19

Example 2 was repeated, except that along the fluid direction, the outlet diameter of the nozzles on the 1st to 11th second spraying pipes of the first spraying pipe was 11.7 mm, and the outlet diameter of the nozzles on the 12th to 14th second spraying pipes of the first spraying pipe was 11.9 mm. After 1 month of operation of the device, the residual ammonia concentration in the tail gas at the reaction outlet was 85 ppm. After 24 months of operation of the device, the residual ammonia concentration in the tail gas at the reaction outlet was 99 ppm. The ratio of acid consumption after 24 months of operation / acid consumption after 1 month of operation was 1.03.

### Example 20

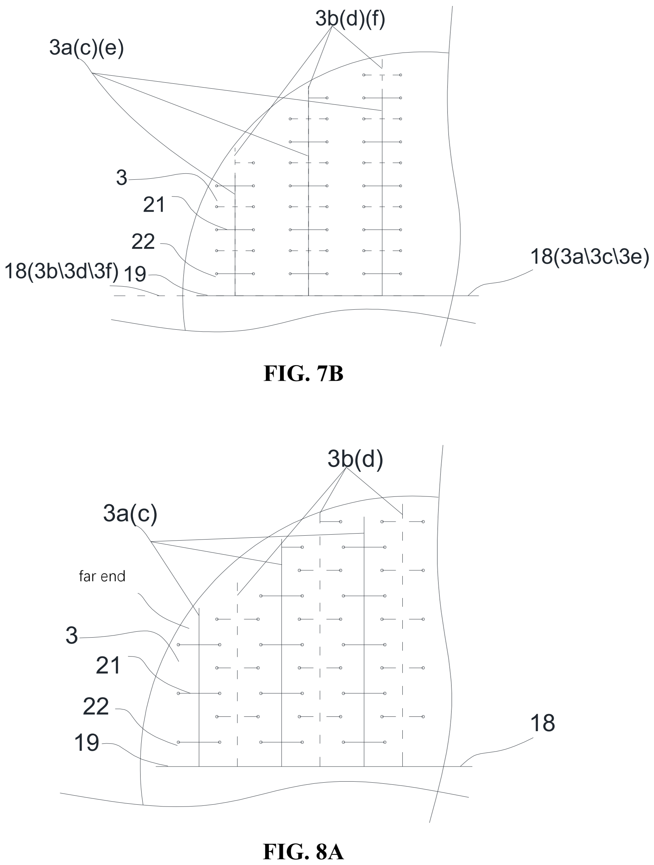

Example 2 was repeated, only except that for the spraying devices 3a, 3b, 3c and 3d, 3e, 3f, the projections of the first spraying pipe and the second spraying pipes coincided, but the projections of the third spraying pipes did not coincide, as shown in Fig.7B. After 1 month of operation of the device, the residual ammonia concentration in the tail gas at the reaction outlet was 64 ppm. After 24 months of operation of the device, the residual ammonia concentration in the tail gas at the reaction outlet was 82 ppm. The ratio of acid consumption after 24 months of operation / acid consumption after 1 month of operation was 1.04.

### Example 21

Example 2 was repeated, except that on each spraying device, on 10 second spraying pipes, two adjacent nozzles on the same side had the same rotating chamber direction, while on the other 4 second spraying pipes, two adjacent nozzles on the same side had opposite rotating directions. The nozzles on the opposite two sides of second spraying pipes of the spraying device had opposite rotating chamber directions. After 1 month of operation of the device, the residual ammonia concentration in the tail gas at the reaction outlet was 81 ppm. After 24 months of operation of the device, the residual ammonia concentration in the tail gas at the reaction outlet was 105 ppm. The ratio of acid consumption after 24 months of operation / acid consumption after 1 month of operation was 1.03.

### Example 22

Example 2 was repeated, except that on each spraying device, on 4 second spraying pipes, two adjacent nozzles on the same side had the same rotating chamber direction, while on the other 10 second spraying pipes, two adjacent nozzles on the same side had opposite rotating directions. The nozzles on the opposite two sides of the second spraying pipes of the spraying device had opposite rotating chamber directions. After 1 month of operation of the device, the residual ammonia concentration in the tail gas at the reaction outlet was 108 ppm. After 24 months of operation of the device, the residual ammonia concentration in the tail gas at the reaction outlet was 125 ppm. The ratio of acid consumption after 24 months of operation / acid consumption after 1 month of operation was 1.04.

### Comparative Example 1

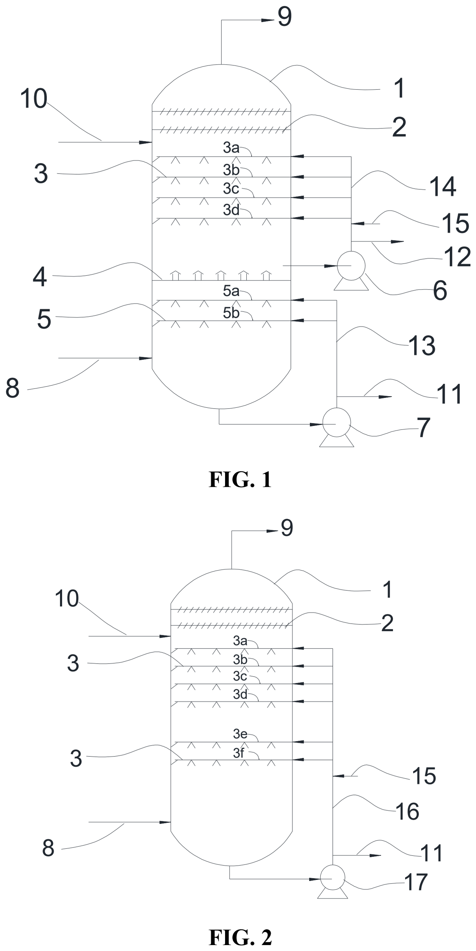

Example 1 was repeated, except that the spraying inlets 18 of spraying devices 3a, 3c and 3b, 3d were on the same side of the ammonia absorption column, i.e., the fluid directions in the first spraying pipes were the same, and the projections of the spraying inlets of spraying devices 3a, 3b, 3c, and 3d substantially coincided (as shown in Fig.1), and the projections of the nozzles at the ends of the third spraying pipes of spraying devices 3a, 3c and those of spraying devices 3b, 3d coincided on the cross-section (as shown in Fig.5). After 1 month of operation of the device, the residual ammonia concentration in the tail gas at the reaction outlet was 85 ppm. After 24 months of operation of the device, the residual ammonia concentration in the tail gas at the reaction outlet was 253 ppm. The ratio of acid consumption after 24 months of operation / acid consumption after 1 month of operation was 1.13.

### Comparative Example 2

Example 2 was repeated, except that the spraying inlets 18 of spraying devices 3a, 3c, 3e and those of spraying devices 3b, 3d, 3f were on the same side of the ammonia absorption column, i.e., the fluid directions in the first spraying pipes were the same, and the projections of the spraying inlets of spraying devices 3a, 3b, 3c, 3d, 3e, 3f substantially coincided (as shown in Fig.2), and the projections of the nozzles at the ends of the third spraying pipes of spraying devices 3a, 3c and those of spraying devices 3b, 3d coincided on the cross-section (as shown in Fig.5). After 1 month of operation of the device, the residual ammonia concentration in the tail gas at the reaction outlet was 73 ppm. After 24 months of operation of the device, the residual ammonia concentration in the tail gas at the reaction outlet was 223 ppm. The ratio of acid consumption after 24 months of operation / acid consumption after 1 month of operation was 1.13.

### Comparative Example 3

Example 2 was repeated, except that the projection angle of the spraying inlets 18 of spraying devices 3a, 3c, 3e and those of spraying devices 3b, 3d, 3f on the cross-section was 30°, i.e., the fluid directions of the corresponding first spraying pipes of the spraying devices were at 30°, such that the projections of the ends of the third spraying pipes of the spraying devices did not coincide, as shown in Fig.8B. After 1 month of operation of the device, the residual ammonia concentration in the tail gas at the reaction outlet was 130 ppm. After 24 months of operation of the device, the residual ammonia concentration in the tail gas at the reaction outlet was 343 ppm. The ratio of acid consumption after 24 months of operation / acid consumption after 1 month of operation was 1.15.

### Comparative Example 4

Example 2 was repeated, except that the projection angle of the spraying inlets 18 of spraying devices 3a, 3c, 3e and those of spraying devices 3b, 3d, 3f on the cross-section was 90°, i.e., the fluid directions of the corresponding first spraying pipes of the spraying devices were at 90°, such that the projections of the ends of the third spraying pipes of the spraying devices did not coincide. After 1 month of operation of the device, the residual ammonia concentration in the tail gas at the reaction outlet was 110 ppm. After 24 months of operation of the device, the residual ammonia concentration in the tail gas at the reaction outlet was 293 ppm. The ratio of acid consumption after 24 months of operation / acid consumption after 1 month of operation was 1.10.

### Comparative Example 5

Example 2 was repeated, except that the projection angle of the spraying inlets 18 of spraying devices 3a, 3c, 3e and those of spraying devices 3b, 3d, 3f on the cross-section was 120°, i.e., the fluid directions of the corresponding first spraying pipes of the spraying devices were at 90°, such that the projections of the ends of the third spraying pipes of the spraying devices did not coincide, as shown in Fig.8B. After 1 month of operation of the device, the residual ammonia concentration in the tail gas at the reaction outlet was 132 ppm. After 24 months of operation of the device, the residual ammonia concentration in the tail gas at the reaction outlet was 353 ppm. The ratio of acid consumption after 24 months of operation / acid consumption after 1 month of operation was 1.15.

## Claims

1. An absorption device, comprising a shell and a plurality of spraying devices arranged in layers along the central axis direction of the absorption device at predetermined vertical spacings, wherein each of the spraying devices independently comprises a spraying liquid inlet, a first spraying pipe in fluid communication with the spraying liquid inlet, a plurality of second spraying pipes in fluid communication with the first spraying pipe and, perpendicularly to the first spraying pipe, arranged along both sides thereof, a plurality of third spraying pipes in fluid communication with the second spraying pipes and, perpendicularly to the second spraying pipes, arranged along both sides thereof, and nozzles located at the ends of the third spraying pipes and in fluid communication therewith, wherein when a cross-section is obtained by cutting the absorption device at a direction perpendicular to the central axis of the absorption device, at least one (preferably all) selected from the group consisting of the first spraying pipe, the second spraying pipes, and the third spraying pipes of one of the plurality of spraying devices and at least one (preferably all) selected from the group consisting of the first spraying pipe, the second spraying pipes, and the third spraying pipes of another of the plurality of spraying devices substantially coincide in terms of the projection on the cross-section, and the angle between the projection on the cross-section of the spraying liquid inlet of the one spraying device and the projection on the cross-section of the spraying liquid inlet of the another spraying device is 180°.

2. The absorption device according to claim 1, wherein on the first spraying pipe, the horizontal spacing between two adjacent second spraying pipes is 640-1300 mm (preferably 700-1200 mm), and/or, on a same second spraying pipe, the horizontal spacing between two adjacent third spraying pipes is 320-650 mm (preferably 350-600 mm), and/or, on two adjacent second spraying pipes, the linear distance M between the end of any one third spraying pipe on one second spraying pipe and the end of any one third spraying pipe on another adjacent second spraying pipe is not less than 320 mm (preferably not less than 350 mm).

3. The absorption device according to claim 1, wherein the nozzles are the same or different from each other, each independently having a spraying liquid ejection rate of 0.5-7.5 t/h (preferably 0.9-6.5 t/h).

4. The absorption device according to claim 1, wherein among all the spraying devices, the angle between the projections on the cross-section of the spraying liquid inlets of any two odd-numbered spraying devices is 0°, the angle between the projections on the cross-section of the spraying liquid inlets of any two even-numbered spraying devices is 0°, and the angle between the projection on the cross-section of the spraying liquid inlet of any odd-numbered spraying device and that of the spraying liquid inlet of any even-numbered spraying device is 180°.

5. The absorption device according to claim 1, wherein all of the nozzles of the one spraying device and all of the nozzles of the another spraying device substantially coincide in terms of the projection on the cross-section, and/or, two nozzles with the substantially coinciding projections have the same spray diameter.

6. The absorption device according to claim 1, wherein the nozzle comprises a nozzle inlet, a rotating chamber, and a nozzle outlet, wherein the rotating chamber is configured such that the spraying liquid fed in through the nozzle inlet leaves the nozzle outlet in a rotating manner after passing through the rotating chamber.

7. The absorption device according to claim 6, wherein the rotating chambers each independently have a diameter of 10.0-55.0 mm (preferably 13.0-45.0 mm), and/or, two nozzles with the substantially coinciding projections have the same rotating direction.

8. The absorption device according to claim 1, wherein on at least one (preferably all) of the second spraying pipes, two adjacent (preferably all) nozzles located on the same side of the second spraying pipe are configured such that the spraying liquid is ejected out with the same rotating direction.

9. The absorption device according to claim 8, wherein all nozzles on the facing sides of two side-by-side adjacent second spraying pipes are configured such that the spraying liquid is ejected out with the opposite rotating directions.

10. The absorption device according to claim 8, wherein on at least one (preferably all) of the second spraying pipes, at least one (preferably all) nozzle located on one side of the second spraying pipe is configured such that the spraying liquid is ejected out with a rotating direction A, while at least one (preferably all) nozzle located on the opposite side of the second spraying pipe is configured such that the spraying liquid is ejected out with a rotating direction B, wherein the rotating direction A is opposite to the rotating direction B.

11. The absorption device according to claim 10, wherein among all the nozzles of the spraying device, the number of nozzles ejecting the spraying liquid with the rotating direction A is equal or substantially equal to the number of nozzles ejecting the spraying liquid with the rotating direction B.

12. The absorption device according to claim 1, wherein the vertical spacing between two adjacent spraying devices (calculated as the vertical spacing of the spraying liquid inlets of the spraying devices) is 650-1350 mm (preferably 750-1200 mm).

13. A process for producing a nitrile, comprising a step of subjecting a hydrocarbon feedstock to an ammoxidation reaction to produce a reaction product containing the nitrile (called as the reaction step), and a step of spraying a spraying liquid to the reaction product to cool the reaction product (called as the cooling step), wherein the spraying liquid is sprayed to the reaction product in an absorption device according to claim 1.

14. The process according to claim 13, wherein in the cooling step, the flow ratio of the spraying liquid to the reaction product is 15-25:1.
